# EUROPEAN PATENT SPECIFICATION

(11) **EP 2 879 666 B1**
(45) Date of publication and mention of the grant of the patent: **08.04.2020**
(21) Application number: 13745311.4
(22) Date of filing: 25.07.2013
(51) Int. Cl.: A61K 9/16, A61K 31/135, A61K 31/445, A61K 31/4545, A61K 31/47, A61K 31/495, A61K 31/573, A61P 37/08

(54) **FREE FLOWING, FROZEN COMPOSITIONS COMPRISING A THERAPEUTIC AGENT**
FREIFLIESSENDE GEFRORENE ZUSAMMENSETZUNGEN MIT EINEM THERAPEUTISCHEN MITTEL
COMPOSITIONS CONGELÉES À ÉCOULEMENT LIBRE COMPRENANT UN AGENT THÉRAPEUTIQUE

(30) Priority: 01.08.2012 US 201261678259 P
(43) Date of publication of application: 10.06.2015
(73) Proprietor: Tavakoli, Zahra, Lexington, KY 40507 (US); Ostertag, Eric, Lexington, KY 40507 (US)
(72) Inventor: Tavakoli, Zahra, Lexington, KY 40507 (US); Ostertag, Eric, Lexington, KY 40507 (US)
(74) Representative: Samson & Partner Patentanwälte mbB
(86) International application number: PCT/US2013/052049
(87) International publication number: WO 2014/022195

(56) References cited:
- EP-A1- 1 080 720
- WO-A1-00/06179
- WO-A1-2005/058474
- WO-A1-2010/009419
- WO-A2-02/060411
- WO-A2-2011/094469
- CA-C- 2 128 244
- US-A- 4 848 094
- US-A- 5 126 156
- US-A- 5 384 129
- US-A- 5 902 606
- US-A1- 2008 138 487
- US-B1- 6 251 684

## Description

### Background of the Invention

Administration of therapeutic agents to pediatric patients is a routine occurance. Unfortunately, treatment compliance can be difficult due to the oftentimes unpleasant taste or odor of therapeutics that must be administered orally. While additives such as flavoring and sugar may be added to help improve flavor and acceptance of orally administered therapeutics, this is often not sufficient to improve palatability such that a child (or even an older consumer) cooperates with administration. A patient's inability or unwillingness to take medication in the manner presecribed can then compromise the health and well being of the child, or, with regard to particularly contagious diseases, may pose a risk to the health of those around them. As such, there is a need for improved methods for administering oral therapeutic agents to children and other patients for whom such administration is necessary.

CA 2 128 244 C discloses a process for the production of pellets containing an active compound (such as prednisone or diphenhydramine), wherein the process comprises (a) dissolving a structure-forming agent in a solvent, (b) dispersing the active compound, and (c) adding the mixture dropwise to liquid nitrogen to produce the pellets.

### Brief Summary of the Invention

The invention is according to the appended claims. In particular, it concerns a frozen, free-flowing composition comprising a therapeutic agent and at least one flavoring agent for use in therapy, wherein said composition comprises beads comprising an ingredient selected from yogurt, milk, coconut milk, soy milk, almond milk, hazelnut milk, oat milk, hemp milk and rice milk, and wherein the composition is obtained by a method comprising the steps of:
a) preparing a composition comprising said therapeutic agent for freezing;
b) dripping said composition into a freezing chamber;
c) freezing said dripping composition into beads;
d) collecting said beads from said freezing chamber; and
e) storing said beads at a temperature sufficient to maintain said beads free-flowing for an extended period of time; wherein said beads are administered after having been stored at a temperature of at least as low as -28.9 °C (-20 °F) and thereafter having been brought to a temperature of from -28.9 °C (-20 °F) to -23.3 °C (-10 °F) before administration such that said beads are free flowing when served; and wherein said beads are administered in an amount such that an effective amount of said therapeutic agent is administered.

### Brief Description of the Drawing

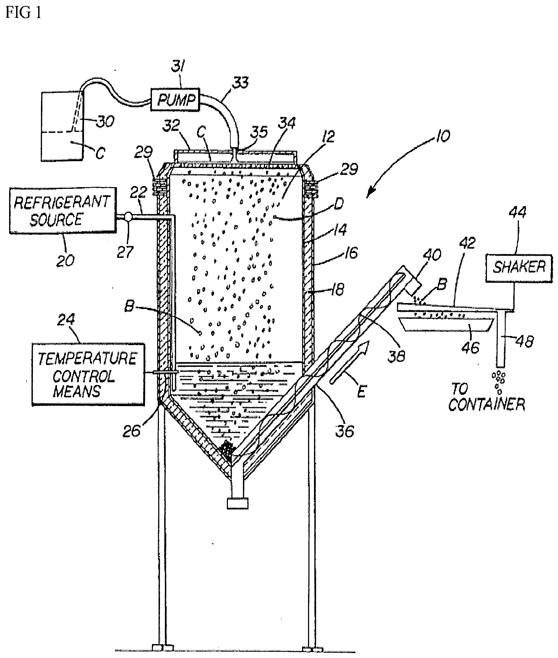

FIG. 1 is a partially schematic cross-sectional view of an exemplary apparatus for preparing a free-flowing, frozen composition as disclosed herein, as taught in US 5,126,156, to Jones, filed September 18, 1991.

### Detailed Description of the Invention

### Definitions

For convenience, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

The articles "a" and "an" are used herein to refer to one or to more than one (i.e., to at least one) of the grammatical object of the article. By way of example, "an element" means one element or more than one element.

As used herein, the term "beads" is intended to encompass droplets that have a substantially smooth, spherical (round or ball shaped) appearance, as well as irregular or odd shaped particles (such as so-called "popcorn" shaped particles).

The term "combination therapy" refers to two or more different active agents which are administered at roughly about the same time (for example, where the active agents are in a single pharmaceutical preparation) or at different times (for example, one agent is administered to the subject before the other). The time difference may range from hours to days, but still constitutes a combination therapy technique.

The term "free" or "substantially free" means the selected composition or method contains or is directed to less than a functional amount of the ingredient or feature, typically less than 0.1% by weight, and also including zero percent by weight, of such ingredient or feature. The nutritional compositions and methods herein may also be "free of" or "substantially free of" any optional or other ingredient or feature described herein provided that the remaining composition still contains the requisite ingredients or features as described herein.

The term "free-flowing" with respect to the disclosed compositions means that the composition is characterized by freedom of movement and is substantially freely pourable, for example, may flow as individual beads, with little or no clumping or sticking to each other, during such pouring. As applied to the instant disclosure, the term refers to the characteristics of the compositions herein, in which the free-flowing compositions comprise a plurality of beads that are substantially able to separate and move relative to one another.

The phrase "effective amount" refers to that amount of a substance that produces some desired local or systemic effect at a reasonable benefit/risk ratio applicable to any treatment. The effective amount of such substance will vary depending upon the subject and disease condition being treated, the weight and age of the subject, the severity of the disease condition, the manner of administration and the like, which can readily be determined by one of ordinary skill in the art

The term "prophylactic" or "therapeutic" treatment is art-recognized and refers to administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The terms "therapeutic agent" refers to a substance, such as a chemical compound or complex that has a measurable beneficial physiological effect on the body, such as a therapeutic effect in treatment of a disease or disorder, when administered in an effective amount. When this term or a particular active agent is specifically identified by name or category, it is understood that such recitation is intended to include the active agent per se, as well as pharmaceutically acceptable, pharmacologically active derivatives thereof, or compounds significantly related thereto, including without limitation, salts, pharmaceutically acceptable salts, N-oxides, prodrugs, active metabolites, isomers, fragments, analogs, solvates hydrates, radioisotopes, etc.

The term "treating" is art-recognized and refers to curing as well as ameliorating at least one symptom of any condition or disease.

### COMPOSITIONS

The composition of the invention is the composition of claim 1. Disclosed are as well free-flowing, frozen compositions that may comprise one or more therapeutic agent and at least one flavoring agent, wherein the composition is frozen and free-flowing. In one aspect, the composition may comprise beads, wherein the beads may have an average diameter of from about 1.27 mm (0.05 inch) to about 12.7 mm (0.5 inch) or less, including 10.16 mm (0.4 inch), 7.62 mm (0.3 inch), 6.35 mm (0.25 inch), 5.08 mm (0.2 inch), 3.81 mm (0.15 inch), and about 2.54 mm (0.1 inch), and ranges including and bordered by these dimensions. In one aspect, the average diameter of the beads is from about 3.175 mm (0.125 inch) to about 7.9375 mm (0.3125 inch), or between about 10 mm and about 2 mm. Exemplary therapeutic agents are described below, and may be present in the composition alone or in combination.

The disclosed frozen composition may have a a temperature of from about -23.3 °C (-10 °F) to about -15 °C (+5 °F) or about -17.8 °C (0 °F). In other aspects, the composition may be stored, for at least about four days, or at least about 14 days, or greater than 14 days, at a temperature at about -23.3 °C (-10 °F) to about -15 °C (+5 °F), wherein the composition is free-flowing during said about four days to about 14 days.

In one aspect, the composition may be dairy based. The compositions may also comprise an ingredient selected from milk, coconut milk, soy milk, almond milk, hazelnut milk, oat milk, hemp milk, rice milk, or a combination thereof. In one aspect, the composition may be substantially dairy free, substantially nut free, substantially gluten free, or a combination thereof. In one aspect, the composition may be substantially free of allergens as commonly recognized in the art, such as soy, wheat, fish, egg, milk, or the like. The composition of the invention comprises beads comprising an ingredient selected from yogurt, milk, coconut milk, soy milk, almond milk, hazelnut milk, oat milk, hemp milk and rice milk.

In one aspect, the composition may comprise a flavoring agent selected from sugar, a sugar substitute, or a combination thereof. In other aspects, the composition may comprise a flavoring agent such as an artificial fruit flavor or the like. The composition of the invention comprises at least one flavoring agent.

In one aspect, the composition may have a total solids content of at least about 25%, at least about 26%, at least about 26.5%, at least about 27%, at least about 27.5%, at least about 28%, at least about 28.5%, at least about 29%, at least about 30%, at least about 31%, at least about 32%, at least about 33%, at least about 34%, at least about 35%, at least about 36%, or at least about 37%, wherein stated percentages are by weight of the weight of the total formulation including water.

In some aspects, the compositions may comprise milkfat. The milkfat content may be from about 6 to about 14%, including about 8-12%, and 7%, 9%, 10%, 11%, and 13% and ranges encompassing and bounded by these values; the nonfat milk solids content is preferably about 4-24%, including about 13-16%, including about 5%, 6%, 7%, 8%, 9%, 10%, 11%, 12%, 14% and 15% and ranges encompassing and bounded by these values; the sugar content is preferably about 2-5%, including about 2.5%, 3%, 3.5%, 4% and 4.5% and ranges encompassing and bounded by these values; the total content of one or more non-sugar or artificial sweeteners is preferably about 0.01-01%, including about 0.1-0.5% and 0.05%, 0.2%, 0.3%, 0.4%, 0.6%, 0.7%, 0.8% and 0.9% and ranges encompassing and bounded by these values; the stabilizer/emulsifier content is preferably about 0.1-4%, including about 0.1%, 2%, and 3% and ranges encompassing and bounded by these values; and the serum solids content is preferably 4-8%, including about 5%, 6%, and 7% and ranges encompassing and bounded by these values. In one preferred embodiment according to Formulation II, the milkfat is approximately 8.4%; the non-fat milk solids is approximately 13%; the sucrose is approximately 3%; the combined stabilizer/emulsifier is approximately 0.3%, the sucralose is approximately 0.02%; and the serum solids are approximately 5.2%; with the remainder being water. All percentages in this paragraph are by weight.

In one aspect, the compositions may comprise nonfat milk solids. Whey solids, including modified whey products, may also be substituted for nonfat milk solids. Egg yolk can also be used as another source of solids. Accordingly, in one aspect, from about 1% to 25%, including 5% to 20% and 10% to 15% of the nonfat milk solids in a formulation comprise whey solids and/or egg yolk solids.

The compositions may further comprise an emulsifier. Emulsifiers can also be included within the various formulations, especially those containing milkfat. Exemplary emulsifiers may include monoglycerides, diglycerides, and polysorbates. Stabilizers may be included within the various formulations. Stabilizers assist in controlling the viscosity of the formulations, with more stabilizer generally providing increased viscosity, especially in those embodiments having lower amounts of fats and solids. The viscosity affects the drip rate of the formulation while it is formed. Stabilizers can include guar, carrageenan, LBG, and/or CMC, and cellulose gum.

In aspects where both stabilizers and emulsifiers are used, the formulations may include a combined stabilizer/emulsifier, and the recited amounts are the combined total of the stabilizer and emulsifier present. The combined stabilizer/emulsifier need not actually be added as a single ingredient when making the formulation; the weights of these two materials are included together because in many embodiments, commercial combined stabilizer/emulsifier formulations are used, which include one or more stabilizers and one or more emulsifiers. Accordingly, the stabilizer/emulsifier may be a commercial or proprietary formulation or it may be a combination or series of one or more stabilizers and/or one or more emulsifiers added to the formulation.

One or more bulking agents may also be added to formulations according to certain embodiments. Bulking agents include high molecular weight polymeric compounds (such as polysaccharides), which add viscosity and bulk to foods. Preferred bulking agents include, but are not limited to polydextrose, dextrans, corn syrup solids, and maltodextrins. In certain preferred embodiments, maltodextrins are used, including, but not limited to, those having a DE of 5, 10, 15, and 20, where DE refers to "dextrose equivalent". In a preferred embodiment, the total amount of bulking agents is 1% to 20% by weight, including 1%-15% by weight, 5%-15% by weight, including 6%, 8%, 10% and 12% by weight. Because bulking agents and stabilizers both contribute to the viscosity of a formulation, formulations containing a bulking agent may or may not include a stabilizer or stabilizer/emulsifier.

The composition of the invention includes one or more flavorings. These include but are not limited to chocolate, strawberry, vanilla, and banana split. The amount of flavoring added may be somewhat small, such that differences in composition are relatively minute such that the flavoring does not substantially affect the storability characteristics of the beads formed from the various formulations.

The compositions may include at least some sugar (sucrose). Sucrose may be present in an amount of from about 2 to about 17% by weight, including 2-8%, 10-17%, 5-15%, about 3%, 4%, 5%, 6%, 7%, 8%, 9%, 10%, 11%, 12%, and 13% and ranges encompassing and bounded by these values. Formulations generally also include some lactose, as it is a natural part of milk, cream, and nonfat milk solids. In a preferred embodiment, the lactose in the formulation is at 2-15% by weight, including 2-8%, 5-10%, 5-15%, about 3%, 4%, 5%, 6%, 7%, 8%, 9%, 10%, 11%, 12%, and 13% and ranges encompassing and bounded by these values. Formulations may include other non-sugar sweeteners in the formulation such as fructose, sugar alcohols also known as polyols, such as erythritol, xylitol, and maltitol, artificial sweeteners including, but not limited to, sucralose, aspartame, and saccharine, and combinations of one or more sweeteners. Because artificial sweeteners are much sweeter than sugar for a given weight, for example, sucralose is about 600 times sweeter than sugar, the amount of sucralose can be very small (e.g. 0.01-0.4% by weight, if present, including about 0.015, 0.02, 0.03, 0.04, 0.05, 0.1, 0.15, 0.2, 0.3 and ranges encompassing and bounded by these values) yet still have effective sweetness. Accordingly, the substitution of artificial sweeteners for sugar can reduce the amount of solids and sucrose in the formulation.

Exemplary non-limiting therapeutic agents:
"Angiotensin I Converting Enzymes (ACE's) and Angiotensin II Receptor Antagonists (ARB's)"; "Angiotensin II receptor antagonists" (ARB's). Angiotensin II receptor antagonists (ARB's) are well known and include peptide compounds and non-peptide compounds. Most angiotensin II receptor antagonists are slightly modified congeners in which agonist activity is attenuated by replacement of phenylalanine in position 8 with some other amino acid; stability can be enhanced by other replacements that slow degeneration in vivo. Examples of angiotensin II receptor antagonists include: peptidic compounds (e.g., saralasin and related analogs); N-substituted imidazole-2-one (U.S. Pat. No. 5,087,634); imidazole acetate derivatives including 2-N-butyl-4-chloro-1-(2-chlorobenzile) imidazole-5-acetic acid (see Long et al., J. Pharmacol. Exp. Ther. 247(1), 1-7 (1988)); 4,5,6,7-tetrahydro-1H-imidazo [4,5-c]pyridine-6-carboxylic acid and analog derivatives (U.S. Pat. No. 4,816,463); N2-tetrazole beta-glucuronide analogs (U.S. Pat. No. 5,085,992); substituted pyrroles, pyrazoles, and tryazoles (U.S. Pat. No. 5,081,127); phenol and heterocyclic derivatives such as 1,3-imidazoles (U.S. Pat. No. 5,073,566); imidazo-fused 7-member ring heterocycles (U.S. Pat. No. 5,064,825); peptides (e.g., U.S. Pat. No. 4,772,684); antibodies to angiotensin II (e.g., U.S. Pat. No. 4,302,386); and aralkyl imidazole compounds such as biphenyl-methyl substituted imidazoles (e.g., EP 253,310, Jan. 20, 1988); ES8891 (N-morpholinoacetyl-(-1-naphthyl)-L-alanyl-(4, thiazolyl)-L-alanyl (35, 45)-4-amino-3-hydroxy-5-cyclo-hexapentanoyl-N-hexylamide, Sankyo Company, Ltd., Tokyo, Japan); SKF108566 (E-alpha-2-[2-butyl-1-(carboxy phenyl) methyl] 1H-imidazole-5-yl[methylane]-2-thiophenepropanoic acid, Smith Kline Beecham Pharmaceuticals, Pa.); Losartan (DUP753/MK954, DuPont Merck Pharmaceutical Company); Remikirin (RO42-5892, F. Hoffman LaRoche A G); alpha-2 agonists (Marion Merrill Dow) and certain non-peptide heterocycles (G. D. Searle and Company). Other non-limiting examples of ARBs include candesartan, eprosartan, irbesartan, losartan, and valsartan.
"Angiotensin converting enzyme" (ACE) inhibitors. ACE inhibitors have been used medically to treat hypertension, congestive heart failure, myocardial infarction and renal disease. Classes of compounds known to be useful as ACE inhibitors include acylmercapto and mercaptoalkanoyl prolines such as captopril (U.S. Pat. No. 4,105,776) and zofenopril (U.S. Pat. No. 4,316,906), carboxyalkyl dipeptides such as enalapril (U.S. Pat. No. 4,374,829), lisinopril (U.S. Pat. No. 4,374,829), quinapril (U.S. Pat. No. 4,344,949), ramipril (U.S. Pat. No. 4,587,258), and perindopril (U.S. Pat. No. 4,508,729), carboxyalkyl dipeptide mimics such as cilazapril (U.S. Pat. No. 4,512,924) and benazapril (U.S. Pat. No. 4,410,520), phosphinylalkanoyl prolines such as fosinopril (U.S. Pat. No. 4,337,201) and trandolopril. Other non-limiting examples of ACE inhibitors may include alacepril, benazepril, captopril, ceronapril, cilazapril, delapril, enalapril, enalaprilat, fosinopril, imidapril, lisinopril, perindopril, quinapril, ramipril, ramiprilat, spirapril, temocapril, trandolapril.

Adrenergic Blockers. Non-limiting examples of adrenergic blockers, both alpha and beta adrenergic blockers, that may be used in the compositions of the present invention include Beta-adrenergic receptor blockers that may include atenolol, acebutolol, alprenolol, befunolol, betaxolol, bunitrolol, carteolol, celiprolol, hedroxalol, indenolol, labetalol, levobunolol, mepindolol, methypranol, metindol, metoprolol, metrizoranolol, oxprenolol, pindolol, propranolol, practolol, sotalolnadolol, tiprenolol, tomalolol, timolol, bupranolol, penbutolol, trimepranol, yohimbine, 2-(3-(1,1-dimethylethyl)-amino-2-hydroxypropoxy)-3-pyridenecarbonitrilHCl, 1-butylamino-3-(2,5-dichlorophenoxy)-2-propanol, 1 -isopropylamino-3 -(4-(2-cyclopropylmethoxyethyl)phenoxy)-2-propanol, 3-isopropylamino-1-(7-methylindan-4-yloxy)-2-butanol, 2-(3-t-butylamino-2-hydroxy-propylthio)-4-(5-carbamoyl-2-thienyl)thiazol, 7-(2-hydroxy-3-t-butylaminpropoxy)phthalide. The above-identified compounds can be used as isomeric mixtures, or in their respective levorotating or dextrorotating form.

Adrenergic Agonists. Non-limiting examples of adrenergic agonists, both alpha and beta adrenergic agonists, that may be used in the compositions of the present invention include adrafinil, adrenalone, albuterol, amidephrine, apraclonidine, bitolterol, budralazine, carbuterol, clenbuterol, clonidine, clorprenaline, clonidine, cyclopentamine, denopamine, detomidine, dimetofrine, dioxethedrine, dipivefrin, dopexamine, ephedrine, epinephrine, etafedrine, ethylnorepinephrine, fenoterol, fenoxazoline, formoterol, guanabenz, guanfacine, hexoprenaline, hydroxyamphetamine, ibopamine, indanazoline, isoetharine, isometheptene, isoproterenol, mabuterol, mephentermine, metaproterenol, metaraminol, metazoline, methoxamine, methylhexaneamine, methoxyphenamine, midodrine, modafinil, moxonidine, naphazoline, norepinephrine norfenefrine, octodrine, octopamine, oxyfedrine, oxymetazoline, phenylephrine hydrochloride, phenylpropanolamine hydrochloride, phenylpropylmethylamine, pholedrine, pirbuterol prenalterol, procaterol, propylhexedrine, protokylol, pseudoephedrine, reproterol, rilmenidine, rimiterol, ritodrine, salmeterol, solterenol, synephrine, talipexole, terbutaline, tetrahydrozoline, tiamenidine, tramazoline, tretoquinol, tuaminoheptane, tulobuterol, tymazoline, tyramine, xamoterol, xylometazoline, and mixtures thereof.

Agents for pheochromocytoma include chemotherapeutics.

Antiangina agents include amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, nitrates (including glyceryl trinitrate (GTN, nitroglycerin, Nitro-Bid), isosorbide-5-mononitrate (5-ISMN, Ismo), amyl nitrate and nicorandil (Icorel)), primidolol, ranolazine hydrochoride, tosifen, verapamil hydrochloride).

Antiarrhythmics. Non-limiting examples of antiarrhythmics that may be used in the compositions of the present invention include acebutolol, acecainide, adenosine, ajmaline, alprenolol, amiodarone, amoproxan, aprindine, aprotinolol, atenolol, azimilide, bevantolol, bidisomide, bretylium tosylate, bucumolol, butetolol, bunaftine, bunitrolol, bupranolol, butidrine hydrochloride, butobendine, capobenic acid, carazolol, carteolol, cifenline, cloranolol, disopyramide, dofetilide, encainide, esmolol, flecainide, hydroquinidine, ibutilide, indecainide, indenolol, ipratropium bromide, lidocaine, lorajmine, lorcainide, meobentine, mexiletine, moricizine, nadoxolol, nifenaolol, oxprenolol, penbutolol, pentisomide, pilsicainide, pindolol, pirmenol, practolol, prajmaline, procainamide hydrochloride, pronethalol, propafenone, propranolol, pyrinoline, quinidine, sematilide, sotalol, talinolol, tilisolol, timolol, tocainide, verapamil, viquidil, xibenolol, and mixtures thereof.

Antiplatelet Agents. Non-limiting examples of antiplatelet agents that may be used in the compositions of the present invention include clopidogrel, dipyridamole, abcixamab, and ticlodipine.

Anticoagulants. Anti-coagulant agents are agents which inhibit the coagulation pathway by impacting negatively upon the production, deposition, cleavage and/or activation of factors essential in the formation of a blood clot. Non-limiting examples of anticoagulants (i.e., coagulation-related therapy) that may be used in the compositions of the present invention include Aggrenox, Agrylin, Amicar, Anturane, Arixtra, Coumadin, Fragmin, Heparin Sodium, Lovenox, Mephyton, Miradon, Persantine, Plavix, Pletal, Ticlid, Trental, Warfarin. Other "anti-coagulant" and/or "fibrinolytic" agents include Plasminogen (to plasmin via interactions of prekallikrein, kininogens, Factors XII, XIIIa, plasminogen proactivator, and tissue plasminogen activator[TPA]) Streptokinase; Urokinase: Anisoylated Plasminogen-Streptokinase Activator Complex; Pro-Urokinase; (Pro-UK); rTPA (alteplase or activase; r denotes recombinant); rPro-UK; Abbokinase; Eminase; Sreptase Anagrelide Hydrochloride; Bivalirudin; Dalteparin Sodium; Danaparoid Sodium; Dazoxiben Hydrochloride; Efegatran Sulfate; Enoxaparin Sodium; Ifetroban; Ifetroban Sodium; Tinzaparin Sodium; retaplase; Trifenagrel; Warfarin; Dextrans.

Still other anti-coagulant agents may include Ancrod; Anticoagulant Citrate Dextrose Solution; Anticoagulant Citrate Phosphate Dextrose Adenine Solution; Anticoagulant Citrate Phosphate Dextrose Solution; Anticoagulant Heparin Solution; Anticoagulant Sodium Citrate Solution; Ardeparin Sodium; Bivalirudin; Bromindione; Dalteparin Sodium; Desirudin; Dicumarol; Heparin Calcium; Heparin Sodium; Lyapolate Sodium; Nafamostat Mesylate; Phenprocoumon; Tinzaparin Sodium.

Inhibitors of platelet function are agents that impair the ability of mature platelets to perform their normal physiological roles (i.e., their normal function). Platelets are normally involved in a number of physiological processes such as adhesion, for example, to cellular and non-cellular entities, aggregation, for example, for the purpose of forming a blood clot, and release of factors such as growth factors (e.g., platelet-derived growth factor (PDGF)) and platelet granular components. One subcategory of platelet function inhibitors are inhibitors of platelet aggregation which are compounds which reduce or halt the ability of platelets to associate physically with themselves or with other cellular and non-cellular components, thereby precluding the ability of a platelet to form a thrombus.

Examples of useful inhibitors of platelet function include acadesine, anagrelide (if given at doses exceeding 10 mg/day), anipamil, argatroban, aspirin, clopidogrel, cyclooxygenase inhibitors such as nonsteroidal anti-inflammatory drugs and the synthetic compound FR-122047, danaparoid sodium, dazoxiben hydrochloride, diadenosine 5',5'"-P1,P4-tetraphosphate (Ap4A) analogs, difibrotide, dilazep dihydrochloride, 1,2- and 1,3-glyceryl dinitrate, dipyridamole, dopamine and 3-methoxytyramine, efegatran sulfate, enoxaparin sodium, glucagon, glycoprotein IIb/IIIa antagonists such as Ro-43-8857 and L-700,462, ifetroban, ifetroban sodium, iloprost, isocarbacyclin methyl ester, isosorbide-5-mononitrate, itazigrel, ketanserin and BM-13.177, lamifiban, lifarizine, molsidomine, nifedipine, oxagrelate, PGE, platelet activating factor antagonists such as lexipafant, prostacyclin (PGI.sub.2), pyrazines, pyridinol carbamate, ReoPro (i.e., abciximab), sulfinpyrazone, synthetic compounds BN-50727, BN-52021, CV-4151, E-5510, FK-409, GU-7, KB-2796, KBT-3022, KC-404, KF-4939, OP-41483, TRK-100, TA-3090, TFC-612 and ZK-36374, 2,4,5,7-tetrathiaoctane, 2,4,5,7-tetrathiaoctane 2,2-dioxide, 2,4,5-trithiahexane, theophyllin pentoxifyllin, thromboxane and thromboxane synthetase inhibitors such as picotamide and sulotroban, ticlopidine, tirofiban, trapidil and ticlopidine, trifenagrel, trilinolein, 3-substituted 5,6-bis(4-methoxyphenyl)-1,2,4-triazines, and antibodies to glycoprotein IIb/IIIa as well as those disclosed in U.S. Pat. No. 5,440,020, and anti-serotonin drugs, Clopridogrel; Sulfinpyrazone; Aspirin; Dipyridamole; Clofibrate; Pyridinol Carbamate; PGE; Glucagon; Antiserotonin drugs; Caffeine; Theophyllin Pentoxifyllin; Ticlopidine.

Antihypertensives. Non-limiting examples of antihypertensives include amlodipine, benidipine, benezepril, candesartan, captopril, darodipine, dilitazem HCl, diazoxide, doxazosin HCl, enalapril, eposartan, losartan mesylate, felodipine, fenoldopam, fosenopril, guanabenz acetate, irbesartan, isradipine, lisinopril, mecamylamine, minoxidil, nicardipine HCl, nifedipine, nimodipine, nisoldipine, phenoxybenzamine HCl, prazosin HCl, quinapril, reserpine, terazosin HCl, telmisartan, and valsartan.

Antilipemic Agents. Non-limiting examples of antilipemic agents include acipimox, aluminum nicotinate, atorvastatin, cholestyramine resin, colestipol, polidexide, beclobrate bezafibrate, ciprofibrate, clinofibrate, clofibrate, clofibric acid, etofibrate, fenofibrate, fluvastatin, gemfibrozil, lovastatin, lysosomal acid lipase, icofibrate, niacin, pirifibrate, pravastatin sodium, ronifibrate, simfibrate, theofibrate, simvastatin, niceritrol, nicoclonate, nicomol. oxiniacic acid, etiroxate, thyopropic acid, thyroxine, acifran, azacosterol, benfluorex, beta-benzalbutyramide, carnitine, chondroitin sulfate clomestrone, detaxtran, dextran sulfate sodium, 5, 8, 11, 14, 17-eicosapentaenoic acid, eritadenine, furazabol, meglutol, melinamide, mytatrienediol, ornithine, gamma-oryzanol, pantethine, pentaerythritol tetraacetate, alpha-phenylbutyramide, pirozadil, probucol, beta-sitosterol, sultosilic acid (piperazine salt), tiadenol, triparanol, xenbucin, and mixtures thereof.

Antidiabetics. Non-limiting examples of antidiabetics that may be used in the compositions of the present invention include biguanides such as buformin, metformin, and phenformin; hormones such as insulin; sulfonylurea derivatives such as acetohexamide, 1-butyl-3-metanilylurea, carbutamide, chlorpropamide, glibornuride, gliclazide, glimepiride, glipizide, gliquidone, glisoxepid, glyburide, glybuthiazole, glybuzole, glyhexamide, glymidine, glypinamide, phenbutamide, tolazamide, tolbutamide, tolcyclamide; HDL agonists; PPAR□ agonists such as thiazolidinediones such as pioglitazone, rosiglitazone, and troglitazone; and others including acarbose, calcium mesoxalate, miglitol, and repaglinide.

Antiinflammatory Agents. Non-limiting examples of antiinflammatory agents that may be used in the compositions of the present invention include Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Glucocorticoids; Zomepirac Sodium.

Calcium Channel Blockers. Non-limiting examples of calcium channel blockers include bepridil, clentiazem, diltiazem, fendiline, gallopamil, mibefradil, prenylamine, semotiadil, terodiline, verapamil, amlodipine, aranidipine, barnidipine, benidipine, cilnidipine, efonidipine, elgodipine, felodipine, isradipine, lacidipine, lercanidpine, manidipine, nicardipine, nifedipine, nilvadipine, nimodipine, nisoldipine, nitrendipine, cinnarizine, flunarizine, lidoflazine, lomerizine, bencyclane, etafenone, fantofarone, perhexiline, and mixtures thereof.

### CETP Inhibitors

### COX-2 Inhibitors

"Direct thrombin inhibitors". Non limiting examples of direct thrombin inhibitors include hirudin, hirugen, hirulog, agatroban, PPACK, and thrombin aptamers.

Diuretics. Non-limiting examples of diuretics include althiazide, bendroflumethiazide, benzthiazide, buthiazide, chlorthalidone, cyclopenthiazide, cyclothiazide, epithiazide, ethiazide, fenquizone, indapamide, hydroflumethiazide, methyclothiazide, meticrane, metolazone, paraflutizide, polythiazide, quinethazone, teclothiazide, trichloromethiazide, chlormerodrin, meralluride, mercamphamide, mercaptomerin sodium, mercumatilin sodium, mercurous chloride, mersalyl, acefylline, 7-morpholinomethyl-theophylline, pamabrom, protheobromine, theobromine, canrenone, oleandrin, spironolactone, acetazolamide, ambuside, azosemide, bumetanide, butazolamide, clopamide, clrexolone, disufamide, ethoxzolamide, furosemide, mefruside, methazolamide, piretanide, torsemide, tripamide, xipamide, aminometradine, amisometradine, amanozine, amiloride, arbutin, chlorazanil, ethacrynic acid, etozolin, hydracarbazine, isosorbide, mannitol, metochalcone, muzolimine, perhexiline, ticrynafen, triamterene, urea, and mixtures thereof.

### Endothelin Receptor Antagonists

HMG-CoA Reductase Inhibitor (Statins). HMG-CoA reductase inhibitors include simvastatin (U.S. Pat. No. 4,444,784), lovastatin (U.S. Pat. No. 4,231,938), pravastatin sodium (U.S. Pat. No. 4,346,227), fluvastatin (U.S. Pat. No. 4,739,073), atorvastatin (U.S. Pat. No. 5,273,995), cerivastatin, and numerous others described in U.S. Pat. Nos. 5,622,985; 5,135,935; 5,356,896; 4,920,109; 5,286,895; 5,262,435; 5,260,332; 5,317,031; 5,283,256; 5,256,689; 5,182,298; 5,369,125; 5,302,604; 5,166,171; 5,202,327; 5,276,021; 5,196,440; 5,091,386; 5,091,378; 4,904,646; 5,385,932; 5,250,435; 5,132,312; 5,130,306; 5,116,870; 5,112,857; 5,102,911; 5,098,931; 5,081,136; 5,025,000; 5,021,453; 5,017,716; 5,001,144; 5,001,128; 4,997,837; 4,996,234; 4,994,494; 4,992,429; 4,970,231; 4,968,693; 4,963,538; 4,957,940; 4,950,675; 4,946,864; 4,946,860; 4,940,800; 4,940,727; 4,939,143; 4,929,620; 4,923,861; 4,906,657; 4,906,624 and 4,897,402. Other non-limiting examples of HMG-CoA reductase inhibitors that may be used in the compositions of the present invention include mevastatin, pitavastatin, rosuvastatin, gemcabene, and probucol.

Inotropic Agents. Non-limiting examples of inotropic agents include acefylline, acetyldigitoxins, 2-amino-4-picoline, amrinone, benfurodil hemisuccinate, bucladesine, camphotamide, convallatoxin, cymarin, denopamine, deslanoside, digitalin, digitalis, digitoxin, digoxin, dobutamine, docarpamine, dopamine, dopexamine, enoximone, erythrophleine, fenalsomine, gitalin, gitoxin, glycocyamine, heptaminol, hydrastinine, ibopamine, lanatosides, loprinine, milrinone, nerifolin, oleandrin, ouabain, oxyfedrine, pimobendan, prenalterol, proscillaridin, resibufogenin, scillaren, scillarenin, strophanthin, sulmazole, theobromine, vesnarinone, xamoterol, and mixtures thereof.

"Renin inhibitors". Renin inhibitors include amino acids and derivatives thereof, peptides and derivatives thereof, and antibodies to renin. Examples of renin inhibitors include urea derivatives of peptides (U.S. Pat. No. 5,116,835); amino acids connected by nonpeptide bonds (U.S. Pat. No. 5,114,937); di- and tri-peptide derivatives (U.S. Pat. No. 5,106,835); amino acids and derivatives thereof (U.S. Pat. Nos. 5,104,869 and 5,095,119); diol sulfonamides and sulfinyls (U.S. Pat. No. 5,098,924); modified peptides (U.S. Pat. No. 5,095,006); peptidyl beta-aminoacyl aminodiol carbamates (U.S. Pat. No. 5,089,471); pyrolimidazolones (U.S. Pat. No. 5,075,451); fluorine and chlorine statine or statone containing peptides (U.S. Pat. No. 5,066,643); peptidyl amino diols (U.S. Pat. Nos. 5,063,208 and 4,845,079); N-morpholino derivatives (U.S. Pat. No. 5,055,466); pepstatin derivatives (U.S. Pat. No. 4,980,283); N-heterocyclic alcohols (U.S. Pat. No. 4,885,292); monoclonal antibodies to renin (U.S. Pat. No. 4,780,401); and a variety of other peptides and analogs thereof (U.S. Pat. Nos. 5,071,837, 5,064,965, 5,063,207, 5,036,054, 5,036,053, 5,034,512, and 4,894,437).

Vasodilators. Non-limiting examples of vasodilators include bencyclane, cinnarizine, citicoline, cyclandelate, ciclonicate, diisopropylamine dichloroacetate, eburnamonine, fasudil, fenoxedil, flunarizine, ibudilast, ifenprodil, isosorbide dinitrate, lomerixine, nafronyl, nicametate, nicergoline, nimodipine, papaverine, pentifylline, tinofedrine, vancamine, vinpocetine, viquidil, amotriphene, bendazol, benfurodil hemisuccinate, benziodarone, chloracizine, chromonar, clobenfurol, clonitrate, cloricromen, dilazep, dipyridamole, droprenilamine, efloxate, erythrityl tetranitrate, etafenone, fendiline, floredil, ganglefence, heart muscle extract, hexestrol bis( diethylaminoethyl ether), hexobendine, hydralazine, itramin tosylate khellin, lidoflazine, mannitol hexanitrate, medibazine, nitroglycerin, isosorbide mononitrate, isosorbide dinitrate, and other nitrates, pentaerythritol tetranitrate, pentrinitrol, perhexiline, pimefylline, prenylamine, propatyl nitrate, pyridofylline, trapidil, tricromyl, trimetazidine, trolnitrate phosphate, visnadine, aluminum nicotinate, bamethan, bencyclane, betahistine, bradykinin, brovincamine, bufeniode, buflomedil, butalamine, cetiedil, ciclonicate, cinepazide, cinnarizine, cyclandelate, diisopropylamine dichloroacetate, eledoisin, fenoxedil, flunazine, hepronicate, ifenprodil, iloprost, inositol niacinate, isoxsuprine, kallidin, kallikrein, moxisylyte, nafronyl, nicametate nicergoline, nicofuranose, nicotinyl alcohol, nylidrin, pentifylline, pentoxifylline, piribedil, prostaglandin El, suloctidil, tolazoline, xanthinol niacinate, and mixtures thereof.

Vasopressors. Non-limiting examples of vasopressors include amezinium methyl sulfate, angiotensin amide, dimetofrine, dopamine, etifelmin, etilefrin, gepefrine, metaraminol, methoxamine, midodrine, norepinephrine, pholedrine, synephrine, and mixtures thereof.
AGE Crosslink Breakers (advanced glycosylation end-product crosslink breakers)
AGE Formation Inhibitors (advanced glycosylation end-product formation inhibitors)
analgesics and anti-inflammatory agents, such as aloxiprin, auranofin, azapropazone, benorylate, capsaicin, celecoxib, diclofenac, diflunisal, etodolac, fenbufen, fenoprofen calcium, flurbiprofen, ibuprofen, indomethacin, ketoprofen, ketorolac, leflunomide, meclofenaminc acid, mefenamic acid, nabumetone, naproxen, oxaprozin, oxyphenbutazone, phenylbutazone, piroxicam, rofecoxib, sulindac, tetrahydrocannabinol, tramadol and tromethamine;
antihelminthics, such as albendazole, bephenium hydroxynaphthoate, cambendazole, dichlorophen, ivermectin, mebendazole, oxamniquine, oxfendazole, oxantel embonate, praziquantel, pyrantel embonate and thiabendazole;
anti-asthma agents, such as zileuton, zafirlukast, terbutaline sulfate, montelukast, and albuterol;
anti-bacterial agents, such as alatrofloxacin, azithromycin, baclofen, benzathine penicillin, cinoxacin, ciprofloxacin HCl, clarithromycin, clofazimine, cloxacillin, demeclocycline, dirithromycin, doxycycline, erythromycin, ethionamide, furazolidone, grepafloxacin, imipenem, levofloxacin, lorefloxacin, moxifloxacin HCl, nalidixic acid, nitrofurantoin, norfloxacin, ofloxacin, rifampicin, rifabutine, rifapentine, sparfloxacin, spiramycin, sulphabenzamide, sulphadoxine, sulphamerazine, ulphacetamide, sulphadiazine, sulphafurazole, sulphamethoxazole, sulphapyridine, tetracycline, trimethoprim, trovafloxacin, and vancomycin;
anti-viral agents, such as abacavir, amprenavir, delavirdine, efavirenz, indinavir, lamivudine, nelfinavir, nevirapine, ritonavir, saquinavir, and stavudine;
anti-depressants, such as amoxapine, bupropion, citalopram, clomipramine, fluoxetine HCl, maprotiline HCl, mianserin HCl, nortriptyline HCl, paroxetine HCl, sertraline HCl, trazodone HCl, trimipramine maleate, and venlafaxine HCl;
anti-epileptics, such as beclamide, carbamazepine, clonazepam, ethotoin, felbamate, fosphenytoin sodium, lamotrigine, methoin, methsuximide, methylphenobarbitone, oxcarbazepine, paramethadione, phenacemide, phenobarbitone, phenytoin, phensuximide, primidone, sulthiame, tiagabine HCl, topiramate, valproic acid, and vigabatrin;
anti-fungal agents, such as amphotericin, butenafine HCl, butoconazole nitrate, clotrimazole, econazole nitrate, fluconazole, flucytosine, griseofulvin, itraconazole, ketoconazole, miconazole, natamycin, nystatin, sulconazole nitrate, oxiconazole, erbinafine HCl, terconazole, tioconazole and undecenoic acid;
anti-gout agents, such as allopurinol, probenecid and sulphinpyrazone;
anti-malarials, such as amodiaquine, chloroquine, chlorproguanil HCl, halofantrine HCl, mefloquine HCl, proguanil HCl, pyrimethamine and quinine sulfate;
anti-migraine agents, such as dihydroergotamine mesylate, ergotamine tartrate, frovatriptan, methysergide maleate, naratriptan HCl, pizotifen maleate, rizatriptan benzoate, sumatriptan succinate, and zolmitriptan;
anti-muscarinic agents, such as atropine, benzhexol HCl, biperiden, ethopropazine HCl, hyoscyamine, mepenzolate bromide, oxyphencyclimine HCl and tropicamide;
anti-neoplastic agents and immunosuppressants, such as aminoglutethimide, amsacrine, azathioprine, bicalutamide, bisantrene, busulfan, camptothecin, capecitabine, chlorambucil, cyclosporin, dacarbazine, ellipticine, estramustine, etoposide, irinotecan, lomustine, melphalan, mercaptopurine, methotrexate, mitomycin, mitotane, mitoxantrone, mofetil mycophenolate, nilutamide, paclitaxel, procarbazine HCl, sirolimus, tacrolimus, tamoxifen citrate, teniposide, testolactone, topotecan HCl, and toremifene citrate;
anti-protozoal agents, such as atovaquone, benznidazole, clioquinol, decoquinate, diiodohydroxyquinoline, diloxanide furoate, dinitolmide, furazolidone, metronidazole, nimorazole, nitrofurazone, ornidazole and tinidazole;
anti-psychotics, such as aripiprazole, clozapine, ziprasidone, haloperidol, molindone, loxapine, thioridazine, molindone, thiothixene, pimozide, fluphenazine, risperidone mesoridazine, quetiapine, trifluoperazine, chlorprothixene, chlorpromazine, perphenazine, trifluopromazine, olanzapine;
anti-thyroid agents, such as carbimazole, paracalcitol, and propylthiouracil;
anti-tussives, such as benzonatate;
anxiolytics, sedatives, hypnotics and neuroleptics, such as alprazolam, amylobarbitone, barbitone, bentazepam, bromazepam, bromperidol, brotizolam, butobarbitone, carbromal, chlordiazepoxide, chlormethiazole, chlorpromazine, chlorprothixene, clonazepam, clobazam, clotiazepam, clozapine, diazepam, droperidol, ethinamate, flunanisone, flunitrazepam, triflupromazine, flupenthixol decanoate, fluphenthixol decanoate, flurazepam, gabapentin, haloperidol, lorazepam, lormetazepam, medazepam, meprobamate, mesoridazine, methaqualone, methylphenidate, midazolam, molindone, nitrazepam, olanzapine, oxazepam, pentobarbitone, perphenazine pimozide, prochlorperazine, pseudoephedrine, quetiapine, risperidone, sertindole, sulpiride, temazepam, thioridazine, triazolam, zolpidem, and zopiclone;
corticosteroids, such as beclomethasone, betamethasone, budesonide, cortisone acetate, desoxymethasone, dexamethasone, fludrocortisone acetate, flunisolide, fluocortolone, fluticasone propionate, hydrocortisone, methylprednisolone, prednisolone, prednisone and triamcinolone;
anti-parkinsonian agents, such as apomorphine, bromocriptine mesylate, lysuride maleate, pramipexole, ropinirole HCl, and tolcapone;
gastro-intestinal agents, such as bisacodyl, cimetidine, cisapride, diphenoxylate HCl, domperidone, famotidine, lanosprazole, loperamide, mesalazine, nizatidine, omeprazole, ondansetron HCL, rabeprazole sodium, ranitidine HCl and sulphasalazine;
keratolytics, such as acetretin, calciprotriene, calcifediol, calcitriol, cholecalciferol, ergocalciferol, etretinate, retinoids, targretin, and tazarotene; lipid regulating agents, such as atorvastatin, bezafibrate, cerivastatin, ciprofibrate, clofibrate, fenofibrate, fluvastatin, gemfibrozil, pravastatin, probucol, and simvastatin; muscle relaxants, such as dantrolene sodium and tizanidine HCl; nutritional agents, such as calcitriol, carotenes, dihydrotachysterol, essential fatty acids, non-essential fatty acids, phytonadiol, vitamin A, vitamin B.sub.2, vitamin D, vitamin E and vitamin K, opioid analgesics, such as codeine, dextropropoxyphene, diamorphine, dihydrocodeine, fentanyl, meptazinol, methadone, morphine, nalbuphine and pentazocine;
sex hormones, such as clomiphene citrate, cortisone acetate, danazol, dehydroepiandrosterone, ethynyl estradiol, finasteride, fludrocortisone, fluoxymesterone, medroxyprogesterone acetate, megestrol acetate, mestranol, methyltestosterone, norethisterone, norgestrel, oestradiol, conjugated estrogens, progesterone, rimexolone, stanozolol, stilbestrol, testosterone and tibolone; stimulants, such as amphetamine, dexamphetamine, dexfenfluramine, fenfluramine and mazindol; drugs for rheumatoid arthritis such as methotrexate, auranofin, aurothioglucose and gold sodium thiomalate; drugs for osteoporosis such as alendronate and raloxifene; local anesthetics; antiherpes drugs such as acyclovir, valacyclovir and famcyclovir; anti-emetics such as ondansetron and granisetron;

Further examples of other active agents which may be suitable include: abecarnil, acamprostate, acavir, acebutolol, aceclofenac, acemetacin, acetaminophen, acetaminosalol, acetanilide, acetohexamide, acetophenazine maleate, acetophenazine, acetoxolone, acetoxypregnenolone, acetretin, acrisorcin, acrivastine, acyclovir, adinazolam, adiphenine hydrochloride, adrafinil, adrenolone, agatroban, ahnitrine, akatinol, alatrofloxacin, albendazole, albuterol, aldioxa, alendronate, alfentanil, alibendol, alitretinoin, allopurinol, allylamines, allylestrenol, alminoprofen, almotriptan, alosetron, aloxiprin, alprazolam, alprenolol, amantadine, ambucetamide, amidephrine, amidinomycin, amiloride, aminoarylcarboxylic acid derivatives, aminoglutethimide, aminoglycosides, aminopentamide, aminopromazine, aminorex, amiodarone, amiphenazole, amiprilose, amisulpride, amitriptyline, amlexanox, amlodipine, amodiaquine, amosulalol, amotriphene, amoxapine, amoxicillin, amphecloral, amphetamine, amphomycin, amphotericin, ampicillin, ampiroxicam, amprenavir, amrinone, amsacrine, amyl nitrate, amylobarbitone, anagestone acetate, anastrozole, andinocillin, androstenediol, androstenediol-17- acetate, androstenediol- 17-benzoate, androstenediol-3-acetate, androstenediol-3-acetate-17- benzoate, androstenedione, androsterone acetate, androsterone benzoate, androsterone propionate, androsterone, angiotensin, anidulatungin, aniracetam, apazone, apicycline, apoatropine, apomorphine, apraclonidine, aprepitant, aprotinin, arbaprostil, ardeparin, aripiprazole, arnikacin, arotinolol, arstiinol, arylacetic acid derivatives, arylalkylamines, arylbutyric acid derivatives, arylcarboxylic acids, arylpiperazines, arylpropionic acid derivatives, aspirin, astemizole, atenolol, atomoxetine, atorvastatin, atovaquone, atropine, auranofn, azapropazone, azathioprine, azelastine, azetazolamide, azithromycin, baclofen, bambuterol, bamethan, barbitone, barnidipine, basalazide, beclamide, beclobrate, befimolol, bemegride, benazepril, bencyclane, bendazac, bendazol, bendroflumethiazide, benethamine penicillin, benexate hydrochloride, benfurodil hemisuccinate, benidipine, benorylate, bentazepam, benzhexol, benziodarone, benznidazole, benzoctamine, benzodiazepine derivatives, benzodiazepine, benzonatate, benzphetamine, benzylmorphine, beperiden, bephenium hydroxynaphthoate, bepridil, betahistine, betamethasone, betaxolol, bevantolol, bevonium methyl sulfate, bexarotene, bezadoxifine, bezafibrate, bialamicol, biapenem, bicalutamide, bietamiverine, bifonazole, binedaline, binifibrate, biricodar, bisacodyl, bisantrene, bisoprolol, bitolterol, bopindolol, boswellic acid, bradykinin, bretylium, bromazepam, bromocriptine, bromperidol, brotizolam, brovincamine, buciclate, bucloxic acid, bucumolol, budralazine, buieniode, bufetolol, buflomedil, bufuralol, bumetanide, bunitrolol, bupranolol, buprenorphine, buproprion, buspirone, busulfan, butalamine, butarphenol, butaverine, butenafine, butenatme, butidrine hydrochloride, butobarbitone, butoconazole nitrate, butoconazole, butofilol, butorphenol, butropium bromide, cabergoline, calcifediol, calcipotriene, calcitriol, caldibine, cambendazole, camioxirole, camostat, camposterol, camptothecin, candesartan, candoxatril, capecitabine, caprate, capsaicin, captopril, carazolol, carbacephems, carbamates, carbamezepine, carbapenems, carbarsone, carbatrol, carbenoxolone, carbimazole, carbromal, carbuterol, carisoprodol, carotenes, caroverine, carteolol, carvedilol, cefaclor, cefazolin, cefbuperazone, cefepime, cefoselis, ceftibuten, celcoxib, celecoxib, celiprolol, cephaeline, cephalosporin C, cephalosporins, cephamycins, cerivastatin, certoparin, cetamolol, cetiedil, cetirizine, cetraxate, chloracizine, chlorambucil, chlorbetamide, chlordantoin, chlordiazepoxide, chlormadinone acetate, chlormethiazole, chloroquine, chlorothiazide, chlorpheniramine, chlorphenoxamide, chlorphentermine, chlorproguanil, chlorpromazine, chlorpropamide, chlorprothixene, chlortetracycline, chlorthalidone, cholecalciferol, chromonar, ciclesonide, ciclonicate, cidofivir, ciglitazone, cilansetron, cilostazol, cimetidine, cimetropium bromide, cinepazet maleate, cinnamedrine, cinnarizine, cinolazepam, cinoxacin, ciprofibrate, ciprofloxacin, cisapride, cisplatin, citalopram, citicoline, clarithromycin, clebopride, clemastine, clenbuterol, clidanac, clinofibrate, clioquinol, clobazam, clobenfurol, clobenzorex, clofazimine, clofibrate, clofibric acid, cloforex, clomipramine, clonazepam, clonidine, clonitrate, clopidogrel, clopirac indomethacin, cloranolol, cloricromen, clorprenaline, clortermine, clotiazepam, clotrimazole, cloxacillin, clozapine, cmepazide, codeine methyl bromide, codeine phosphate, codeine sulfate, codeine, colloidal bismuth subcitrate, cromafiban, cromolyn, cropropamide, crotethamide, curcumin, cyclandelate, cyclarbamate, cyclazocine, cyclexedrine, cyclizine, cyclobenzaprine, cyclodrine, cyclonium iodide, cyclopentamine, cyclosporin, cypionate, cyproheptadine, cyproterone acetate, cytarabine, dacarbazine, dalfopristine, dantrolene sodium, dapiprazole, darodipine, decanoate, decitabine, decoquinate, dehydroemetine, delavirdine, del aviridine, demeclo cycline, denopamine, deramciclone, descitalopram, desipramine, desloratadine, 3- ketodesogeskel, desomorphine, desoxymethasone, detomidine, dexamphetamine, dexanabinol, dexchlorpheniramine, dexfenfluramine, dexmethylphenidate, dexrazoxane, dextroamphetamine sulfate, dextroamphetamine, dextropropoxyphene, DHEA, diacetate, diamorphine, diazemine, diazepam, diaziquinone, diazoxide, dibromopropamidine, dichlorophen, diclofenac, dicoumarol, didanosine, dideoxyadenosine, diethylpropion, difemerine, difenamizole, diflunisal, digitoxin, digoxin, dihidroergotamine, dihydrocodeine, diLydrocodeinone enol acetate, dihydroergotamine mesylate, dihydroergotamine, dihydrogesterone, dihydromorphine, dihydropyridine derivatives, dihydrostreptomycin, dihydrotachysterol, dihydroxyaluminum acetylsalicylate, diiodohydroxyquinoline, diisopromine, dilazep, dilevalol, dilitazem, diloxanide furoate, diloxanide, diltiazem, dimefline, dimenhydrinate, dimethisterone, dimetotrine, dimorpholamine, dinitolmide, dioxaphetyl butyrate, dioxethedrine, diphemethoxidine, diphenhydramine, diphenoxylate, diphetarsone, dipivefrin, diponium bromide, dipyridamole, dirithromycin, disopyramide, divalproex sodium, dofetilide, domperidone, donezepil, dopexamine, dopradil, dosmalfate, doxapram, doxazosin, doxefazepam, doxepin, doxycycline, drofenine, dromostanolone propionate, dromostanolone, dronabinol, droperidol, droprenilamine, d-threo -methylphenidate, duloxetine, ebrotidine, eburnamonine, ecabet, ecenofloxacin, econazole nitrate, edavarone, edoxudine, efavirenz, effivarenz, efloxate, eledoisin, eletriptan, elgodipine, ellipticine, emepronium bromide, emetine, enalapril, enanthate, encainide, enlopitat, enoximone, enprostil, entacapone, epanolol, ephedrine, epinastine, epinephrine, epirubicin, epleronone, eposartan, ergocalciferol, ergoloid mesylates, ergotamine, ertapenum, erythromycin, erytlirityl tetranitrate, esaprazole, escitalopram, esmolol, esomeprazole, esonarimod, estazolam, estradiol benzoate, estramustine, eskiol succinate, estrone acetate, estrone sulfate, etafedrine, etafenone, ethacrynic acid, ethamivan, ethinamate, ethinyleskadiol 3-acetate, ethinyleskadiol 3-benzoate, ethinylestradiol, ethionamide, ethisterone (17a-ethinyltestosterone), ethopropazine, ethotoin, ethoxyphenamine, ethylestrenol, ethylmorphine, ethylnorepinephrine, ethynodiol diacetate, etodolac, etofibrate, etoposide, etoricoxib, etretinate, everolimus, exalamide, examestane, examorelin, ezemitibe, falecalcitriol, famciclovir, famotidine, fantofarone, farapenum, farglitazar, fasudil, felbamate, felodipine, fenalamide, fenbulen, fenbutrazate, fendiline, fenfluramine, fenoldopam, fenoprofen, fenoterol, fenoverine, fenoxazoline, fenoxedil, fenpiprane, fenproporex, fenspiride, fentanyl, fexofenadine, flavoxate, flecainide, flopropione, floredil, floxuridine, fluconazole, flucytosine, fludarabine, fludiazopam, fludrocortisone, flulenamic acid, flunanisone, flunarizine, flunisolide, flunitrazepam, fluocortolone, fluoxetine, flupenthixol decanoate, fluphenazine decanoate, fluphenazine enanthate, fluphenazine, fluproquazone, flurazepam, flurbiprofen, flurogestone acetate, fluticasone propionate, fluvastatin, fluvoxamine, fominoben, formoterol, foscarnet, foscarnet, fosinopril, fosphenytoin, frovatirptan, fudosteine, fumagillin, furazolidone, furazolidone, furfurylmethyl amphetamine, furosemide, gabapentin, gabexate, gaboxadol, galanthamine, gallopamil, gammaparin, gancyclovir, ganglefene, gefarnate, gemcitabine, gemfibrozil, gepirone, gestadene, ghrelin, glatiramer, glaucarubin, glibenclamide, gliclazide, glimepiride, glipizide, gluconic acid, glutamicacid, glyburide, glyceryl trinitrate, glymepiride, granisetron, grepafloxacin, griseofulvin, guaiazulene, guanabenz, guanfacine, halofankine, haloperidol decanoate, haloperidol, haloxazolam, hepronicate, heptanoate, hexobendine, hexoprenaline, hydramitrazine, hydrazides, hydro chlorothi azide, hydrocodone, hydrocortisone, hydromorphone, hydroxyamphetamine, hydroxymethylprogesterone acetate, hydroxymethylprogesterone, hydroxyprogesterone acetate, hydroxyprogesterone caproate, hydroxyprogesterone, hymecromone, hyoscyamine, ibopamine, ibudilast, ibutenac, ibuprofen, ibutilide, idoxuridine, ifenprodil, igmesine, iloprost, imatinib, imidapril, imidazoles, imipenem, imipramine, imolamine, incadronic acid pergolide, indanazoline, indenolol, indinavir, indomethacin, indoramin, inosinepranobex, inositol niacinate, iodoquinol, ipidracine, iproniazid, irbesartan, irinotecan, irsogladine, isobutyrate, isocaprate esters, isoetharine, isometheptene, isoproterenol, isosorbide dinitrate, isosorbide mononitrate, isosorbide dinitrate, isoxsuprine, isradipine, itasetron, itraconazole, itramintosylate, ivermectin, kallidin, kallikrein, kanamycin, ketamine, ketoconazole, ketoprofen, ketorolac, ketotifen, labetalol, lafutidine, lamifiban, lamivudine, lamotrigine, lanatoside c, lansoprazole, lasofoxifene, leflunomide, leminoprazole, lercanadipine, lesopitron, letrozole, leucovorin, levalbuterol, levallorphan, levetiracetam, levetriacetam, levobunolol, levodopa, levofloxacin, levophacetoperane, levorphanol, lidocaine, lidoflazine, lifibrol, limaprost, linezolid, lintitript, liranaftate, lisinopril, lisuride, lobeline, lobucavir, lodoxamide, lomefloxacin, lomerizine, lomustine, loperamide, lopinavir, loprazolam, loracarbef, loratadine, lorazepam, lorefloxacin, lormetazepam, losartan, lovasatain, lovastatin, loxapine succinate, loxapine, 1-threo methylphenidate, lumiracoxib, lysine acetylsalicylate, lysozyme, lysuride, mabuterol, mafenide, magnesium acetylsalicylate, malgramostin, mannitol hexanitrate, maprotiline, mazindol, mebendazole, meclizine, meclofenamic acid, mecloxaminepentapiperide, medazepam, medibazine, medigoxin, medrogestone, medroxyprogesterone acetate, mefenamic acid, mefenorex, mefloquin, mefloquine, megestrol acetate, melengestrol acetate, melphalan, mematine, mepenzolate bromide, meperidine, mephenoxalone, mephentermine, mepindolol, mepixanox, meprobamate, meptazinol, mercaptopurine, merropenum, mesalamine, mesalazine, mesoridazine besylate, mesoridazine, metaclazepam, metamfepramone, metampicillin, metaproterenol, metaraminol, methacycline, methadone hydrochloride, methadone, methamphetamine, methaqualone, metharnphetamine, methoin, methotrexate, methoxamine, methsuximide, methylhexaneamine, methylphenidate d-threo-methylphenidate, methylphenidate, methylphenobarbitone, methylprednisolone, methysergide, metiazinic acid, metizoline, metoclopramide, metolazone, metoprolol, metoxalone, metripranolol, metronidazole, mexiletine, mexilitene, metaxalone, mianserin, mibefradil, miconazole, midazolam, midodrine, miglitol, milnacipran, milrinone, minoxidil, mirtazapine, misoprostol, mitomycin, mitotane, mitoxantrone, mizolastine, modafinil, mofebutazone, mofetil, molindone hydrochloride, molindone, molsidomine, monatepil, montelukast, monteplase, moprolol, moricizine, morphine hydrochloride, morphine sulfate, morphine, morpholine salicylate, mosapramine, moxifloxacin, moxisylvyte, moxonidine, mycophenolate, nabumetone, nadolol, nadoxolol, nadroparin, nafamostat, nafronyl, naftopidil, nalbuphine, nalidixic acid, nalmefene, nalorphine, naloxone, naltrexone, nandrolone benzoate, nandrolone cyclohexanecarboxylate, nandrolone cyclohexane-propionate, nandrolone decanoate, nandrolone furylpropionate, nandrolone phenpropionate, naphazoline, naproxen, naratriptan, natamycin, nateglinide, nebivalol, nedocromil, nefazodone, nefopam, nelfinavir, nemonapride, neomycin undecylenate, neomycin, neokofin, nesiritide, n- ethylamphetamine, nevibulol, nevirapine, nexopamil, nicametate, nicardipine, nicergoline, nicofibrate, nicofuranose, nicomorphine, nicorandil, nicotinyl alcohol, nicoumalone, nifedipine, nifenalol, nikethamide, nilutamide, nilvadipine, nimodipine, nimorazole, nipradilol, nisoldipine, nitisonone, nitrazepam, nitrofurantoin, nitrofurazone, nitroglycerin, nizatidine, norastemizole, norepinephrine, norethynodrel, norfenefrine, norfloxacin, norgestimate, norgeskel, norgestrienone, normethadone, normethisterone, normorphine, norpseudoophedrine, nortriptyline, novantrone, nylidrin, nystatin, octamylamine, octodrine, octopamine, ofloxacin, olanzapine, olanzapine, olapatadine, olmesartan, olopatidine, olsalazine, omapatrilat, omeprazole, ondasetron, opium, oprevelkin, orlistat, ornidazole, ornoprostil, oseltamivir, oxaliplatin, oxamniquine, oxandrolone, oxantel embonate, oxaprozin, oxatomide pemirolast, oxatomide, oxazopam, oxcarbazepine, oxfendazole, oxiconazole, oxiracetam, oxolinicacid, oxprenol, oxycodone, oxyfedrine, oxymetazoline, oxymorphone, oxyphenbutazone, oxyphencyclimine, oxyprenolol, ozagrel, paclitaxel, palonosetron, pantoprazole, papaverine, paracalcitol, paramethadione, parecoxib, pariprazole, paromomycin, paroxetine, parsalmide, pazinaclone, pemoline, penbutolol, penciclovir, penicillin G benzathine, penicillin G procaine, penicillin V, penicillins, pentaerythritol tetranitrate, pentapiperide, pentazocine, pentifylline, pentigetide, pentobarbitone, pentorex, pentoxifylline, pentrinitrol, perbuterol, perenzepine, pergolide, perhexiline, perindopril erbumine, perospone, perphenazine pimozide, perphenazine, phanquinone, phenacemide, phenacetin, phenazopyridine, phencarbamide, phendimetrazine, phenelzine, phenindione, phenmetrazine, phenobarbitone, phenoperidine, phenothiazines, phenoxybenzamine, phensuximide, phentermine, phentolamine, phenylsalicylate, phenylacetate, phenylbutazone, phenylephrinehydrochloride, phenylpropanolamine hydrochloride, phenylpropanolaminehydrochloride, phenylpropyl-methylamine, phenytoin, phloroglucinol, pholedrine, physostigwine salicylate, physostigmine, phytonadiol, phytosterols, piapenum, picilorex, piclamilast, picrotoxin, picumast, pifarnine, pilsicainide, pimagedine, pimeclone, pimecrolimus, pimefylline, pimozide, pinaverium bromide, pindolol, pioglitazone, piperacillin, piperazine estrone sulfate, piperazine derivatives, piperilate, piracetam, pirbuterol, pirenzepine, piribedil, pirifibrate, piroxicam, pitavastatin, pizotyline, plaunotol, polaprezinc, polybenzarsol, polyestrol phosphate, practolol, pralnacasan, pramipexole, pranlukast, pravastatin, prazepam, praziquantel, prazosin, pregabalin, prenalterol, prenylamine, pridinol, prifinium bromide, primidone, primipramine, probenecid, probucol, procainamide, procarbazine, procaterol, prochlorperazine, proguanil, pronethalol, propafenone, propamidine, propatyl nitrate, propentoffyline, propionate, propiram, propoxyphene, propranolol, propylhexedrine, propylthiouracil, protokylol, protriptyline, proxazole, pseudoephedrine, purines, pyrantel embonate, pyrazoles, pyrazolones, pyridofylline, pyrimethamine, pyrimidines, pyrrolidones, quazepam, quetiapine, quetuapine, quinagolide, quinapril, quinestrol, quinfamide, quinidine, quinine sulfate, quinolones, quinupritin, rabalzotan, rabeprazole sodium, rabeprazole, racefimine, ramahroban, ramipril, ranitidine, ranolazine, ransoprazole, rasagiline, rebamipide, refludan, repaglinide, repinotan, repirinast, reproterol, reserpine, retinoids, ribavirin, rifabutine, rifampicin, rifapentine, rilmenidine, riluzole, rimantadine, rimiterol, rioprostil, risperidone, ritanovir, ritapentine, ritipenem, ritodrine, ritonavir, rivastigrnine, rizatriptan, rociverine, rofecoxib, rohypnol, rolipram, romoxipride, ronifibrate, ropinirole, ropivacaine, rosaprostol, rosiglitazone, rosuvastatin, rotinolol, rotraxate, roxatidine acetate, roxindole, rubitecan, salacetamide, salicin, salicylamide, salicylic acid derivatives, salmeterol, saquinavir, saquinavir, scopolamine, secnidazole, selegiline, semotiadil, sertindole, sertraline, sibutramine, sildenafil, simBibrate, simvastatin, siramesine, sirolimus, sitaxsentan, sofalcone, somotiadil, sorivudine, sotalol, soterenol, sparfloxacin, spasmolytol, spectinomycin, spiramycin, spizofurone, stavudine, streptomycin, succinylsulfathiazole, sucralfate, sufentanil, sulconazole nitrate, sulfacetamide, sulfadiazine, sulfaloxicacid, sulfarside, sulfmalol, sulindac, suloctidil, sulphabenzamide, sulphacetamide, sulphadiazine, sulphadoxine, sulphafurazole, sulphamerazine, sulphamethoxazole, sulphapyridine, sulphasalazine, sulphinpyrazone, sulpiride, sulthiame, sultopride, sulbroponium, sumanirole, sumahriptan, sunepihon, superoxide dismutase, suplatast, suramin sodium, synephrine, tacrine, tacrolimus, tacrolimus, tadalafil, talinolol, talipexole, tamoxifen, tamsulosin, targretin, tazanolast, tazarotene, tazobactum, tecastimezole, teclozan, tedisamil, tegaserod, telenzepine, telmisartan, temazepam, teniposide, teprenone, terazosin, terbenafine, terbinafine, terbutaline sulfate, terbutaline, terconazole, terfenadine, terodiline, terofenamate, tertatolol, testolactone, 4-dihydrotestosterone, tetracyclics, tetracycline, tetrahydrocannabinol, tehrahydrozoline, thalidomide, theofibrate, thiabendazole, thiazinecarboxamides, thiocarbamates, thiocarbamizine, thiocarbarsone, thioridazine, thiothixene, tiagabine, tiamenidine, tianeptine, tiaprofenic acid, tiaramide, ticlopidine, tigloidine, tilisolol, timolol, tinidazole, tinofedrine, tinzaparin, tioconazole, tipranavir, tirapazamine, tirofiban, tiropramide, titanicene, tizanadine, tizanidine, tizinadine, tocainide, tolazamide, tolazoline, tolbutamide, tolcapone, tolciclate, tolfenamic acid, toliprolol, tolteridine, tolterodine, tonaberstat, topiramate, topotecan, torasemide, toremifene cibrate, toremifene, tosufloxacin, tramadol, tramazoline, trandolapril, tranilast, tranylcypromine, trapidil, traxanox, trazodone, tretoquinol, triacetin, triamcinolone, triampterine, triamterene, triazolam, trifluoperazine hydrochloride, trifluoperazine, triflupromazine, trihexyphenidyl, trimazosin, trimebutine, trimetazidine, trimethoprim, trimgestone, trimipramine, trimoprostil, trithiozine, troglitazone, trolnibrate phosphate, tromethamine, tropicamide, trovafloxacin, troxipide, tuaminoheptane, tulobuterol, tymazoline, tyramine, undecanoate, undecanoic acid, urinastatin, valacyclovir, valdecoxib, valerate, valganciclovir, valproic acid, valsartan, vancomycin, vardenafil, venlafaxine, venorelbine, verapamil, verapimil, vidarabine, vigabakin, vincamine, vinpocetine, viomycin, viquidil, visnadine, vitamin a derivatives, vitamin a, vitamin b2, vitamin d, vitamin e, vitamin k, voglibose, voriconazole, xaliproden, xamoterol, xanthinol niacinate, xenytropium bromide, xibenolol, ximelagatran, xylometazoline, yohimbine, zacopride, zafirlukast, zalcitabine, zaleplon, zanamivir, zatebradine, ziconotide, zidovudine, zileuton, zimeldine, zinc propionate, ziprasidone, zolimidine, zolmitriptan, zolpidem, zonisamide, zopiclone, and mixtures thereof.

Administration of the compositions of the present invention will be in an amount sufficient to achieve a therapeutic effect as recognized by one of ordinary skill in the art.

The dosage of any compositions of the present invention will vary depending on the symptoms, age and body weight of the patient, the nature and severity of the disorder to be treated or prevented, the route of administration, and the form of the subject composition. Any of the subject formulations may be administered in a single dose or in divided doses. Dosages for the compositions of the present invention may be readily determined by techniques known to those of skill in the art or as taught herein.

### Methods of Making Compositions

The composition of the invention is prepared according to claim 1.

Also disclosed are methods of preparing and storing a frozen, free-flowing composition comprising a therapeutically active agent, that may comprise the steps of: preparing a composition comprising a therapeutic agent for freezing; dripping the composition into a freezing chamber; freezing the dripping composition into beads; storing the beads at a temperature at least as low as -28.9 °C (-20 °F) so as to maintain the beads free-flowing for an extended period of time; and bringing the beads to a temperature between substantially -23.3 °C (-10 °F) and -28.9 °C (-20 °F) prior to administration to a patient in need thereof; wherein the beads are administered at a temperature between about -23.3 °C (-10 °F) and about -28.9 °C (-20 °F) such that said beads are free flowing when served; and wherein the beads are administered in an amount such that a therapeutically effective amount of the therapeutic agent is administered. The temperature within the freezing chamber may be maintained below about -162.2 °C (-260 °F).

The dripping step may include the step of adding said composition into a tray having a series of apertures through which said composition drips into said freezing chamber, said apertures having diameters of predetermined sizes. The beads may be collected from the freezing chamber by utilizing an auger, which may be positioned at approximately 45° relative to horizontal and includes flights having a diameter of about 3.81 (1.5) to about 8.89 cm (3.5 inches). The collecting step may include the step of rotating said auger at about 10 to about 100 revolutions per minute. The method may further include the step of sifting beads collected from the freezing chamber. Beads having a diameter larger than about 2 mm may be recovered during sifting and placed within a container.

Liquid nitrogen may be utilized to freeze the composition. The container may be left open for a sufficient period of time to allow any nitrogen retained in or on the beads during freezing to vaporize. The container may then be sealed, and the beads may be stored at a temperature at least as low as -34.44 °C (-30 °F), or at a temperature of about -34.4° (-30°) to -40 °C (-40 °F). The beads may be brought to a temperature of about -26.1 °C (-15 °F), prior to serving.

In other aspects, the compositions may be made according to Jones, US 2008/0138487, Serial No. 11/701,624 filed February 2, 2007, wherein methods are taught for the manufacture of free flowing frozen compositions that remain free flowing when stored in a typical retail grocery or home freezing environment.

### Exemplary Methods

The initial step of the method may be the preparing of a composition containing a one or more therapeutic agents as described above for freezing.

Once prepared, the composition may be slowly dripped into a freezing chamber. The composition containing a therapeutic agent may be added into a tray as by pouring from a pitcher or a supply container. A pump adapted for this purpose is manufactured by Cole-Parmer Instrument Company under the trademark MASTERFLEX. The tray may include a series of apertures through which the composition containing a therapeutic agent drips into a freezing chamber. In one aspect, the aperature may have a diameter from about 1.27 (0.05) to about 12.7 mm (0.5 inch), or from about 1.78 (0.07) to about 17.8 mm (0.7 inch), or from about 3.175 (0.125) to about 7.9375 mm (0.3125 inch). The size of the apertures may be varied to provide specific sized droplets depending upon the thickness of the composition. As the droplets fall through the freezing chamber, rapid, almost simultaneous, freezing takes place and small beads of frozen product containing a therapeutic agent may be formed.

In one aspect, the freezing chamber may be maintained at a temperature below about -162.2 °C (-260 °F). This can be accomplished, for example, by utilizing liquid nitrogen as a refrigerant. More specifically, the droplets may fall downwardly in the freezing chamber through the vaporized refrigerant until they actually contact the liquid refrigerant in the bottom of the chamber. This procedure allows the maximum extraction of available refrigeration from the liquid nitrogen. The rapid freezing that results may lead to the formation of smaller ice crystals in the product. Consequently, the product is smoother, creamier and exhibits better flavor, which may serve to increase the consumer appeal of the product containing a therapeutic agent.

After the frozen beads of product are formed, the beads may be collected from the freezing chamber. This may be accomplished by utilizing an auger. The auger may be positioned at an angle of approximately 45° with respect to the horizontal. The auger may also include flights having a diameter of about 3.81 (1.5) to about : 8.89 cm (3.5 inches). When such an auger is rotated at about 10 to about 100 revolutions per minute, depending on the size of the flights, the individual frozen beads may be recovered with a minimum loss of refrigerant from the freezing chamber which may benefit overall processing efficiency.

The beads collected from the freezing chamber may then be sifted. In one aspect, the beads may be placed in a sieve which is shaken so that beads having a specified diameter are recovered. In one aspect, the beads having a diameter greater than about 2 mm are recovered for further processing and packaging. Smaller beads may then be melted and reprocessed as described above or used in other products that require or can use the tiny frozen beads.

The large beads, which may be between about 10 mm and about 2 mm in diameter, recovered following the sifting step may then be placed within a container. This container may be maintained open for a sufficient period of time, such as, for example, about 1 to about 10 minutes, to allow any residual refrigerant retained in or on the beads during freezing to vaporize. Following this, the container may be sealed for storage. The container may then be placed in a freezer. The temperature within the freezer may be maintained at least as low as about -28.9 °C (-20 °F) or between about -34.4 °C (-30 °F) and about -40 °C (-40 °F), if the product is to be stored for periods of greater than approximately 30 hours. This is necessary to ensure that the individual beads remain free-flowing and that no large ice crystals are formed during thaw/refrigeration cycles.

Prior to administering the composition, the beads may be brought to a temperature of about -28.9 °C (-20 °F) or above. In one aspect, the beads may be maintained at a temperature of about -26.1 °C (-15 °F) for no longer than approximately 30 hours prior to serving. If maintained at this or a warmer temperature for a longer period of time, the beads may become tacky and begin sticking together. Thus, the free-flowing characteristic may be lost. As such, storage at a temperature of about -26.1 °C (-15 °F) for longer than about 30 hours may be avoided. For certain compositions, however, it should be recognized that the critical time may be as short as about ten to about twelve hours.

Reference is now made to FIG. 1 showing an apparatus 10 that may be utilized to produce free-flowing, frozen compositions containing a therapeutic agent in accordance with the method disclosed herein. Apparatus 10 is merely being described as an example of one type of apparatus designed for this purpose. Other designs may be utilized in accordance with the present method to produce the disclosed free-flowing, frozen compositions.

As shown, the apparatus 10 includes a freezing chamber 12 having an inner wall 14 and outer wall 16. Both the walls may be constructed of stainless steel to provide both strength and corrosion resistance. A thick layer of thermal insulating material 18 is provided between the walls to improve the efficiency of the freezing chamber by reducing the thermal transfer through the walls 14, 16 between the interior of the chamber 12 and the ambient environment.

The chamber 12 is chilled by the direct addition of refrigerant from a refrigerant source 20 through the delivery line 22. A number of different refrigerants can be utilized. In one aspect, the refrigerant may be liquid nitrogen. This material is readily available, relatively inexpensive and relatively inert to food products. It is also sufficiently cold to provide for relatively rapid freezing of the product. As such, it is particularly adapted for utilization in the processing of free-flowing, compositions containing a therapeutic agent.

The temperature of the freezing chamber as well as the level of liquid refrigerant may be maintained within a specified range through the utilization of a temperature control means 24 such as a thermostat as is known in the art. More specifically, the temperature control means 24 may be connected to a thermocouple 26. The thermocouple 26 may be positioned to extend into the freezing chamber 12 at a selected height between, for example, 10.2 (4) to 45.8 cm (18 inches) above the bottom of the chamber to sense the temperature within the chamber. Where, for example, liquid nitrogen is utilized as the refrigerant, the thermostat is set to maintain the temperature within the chamber 12 at the thermocouple 26 between approximately -184.4° (-300°) to -195.6 °C (-320 °F). The positioning of the thermocouple 26 some 10.2 (4) to 45.8 cm (18 inches) above the bottom of the chamber 12 may provide the necessary reservoir of refrigerant to quick freeze the droplets of the compositions. The ultra-low temperature of the refrigerant limits the formation of ice crystals in the product as it is frozen. Advantageously, by reducing the overall size of the ice crystals being formed, the resulting frozen product may have a richer, creamier texture and exhibits a better, overall flavor.

For example, when the temperature within the chamber 12 at the thermocouple 26 rises above the set range of operation (i.e. -184.4° (-300°) to -195.6 °C (-320 °F)), this is an indication that the level of liquid refrigerant has fallen below the thermocouple. As a result of the operation of the temperature control means 24, a valve 27 is then opened to allow delivery of liquid nitrogen from the source 20 through the line 22 to the chamber 12. Once the liquid refrigerant level within the chamber 12 reaches and contacts the thermocouple 26, the desired level of liquid refrigerant for freezing the composition is restored and the valve 27 is closed.

Alternative temperature or level control systems may be utilized. For example, a number of thermocouples 26 may be positioned at various heights within the chamber 12. The thermocouple 26 at the desired liquid refrigerant level to be maintained is then selected and utilized as described above. In another alternative, a liquid nitrogen level controller such as manufactured and marketed by Minnesota Valley Engineering, Inc. under the trademark CRYO-MED (Model LL-450) may be utilized.

Vents 29 are provided in the walls 14, 16 near the top of the freezing chamber 12. These vents 29 serve to release rising nitrogen vapor from the chamber 12 and prevent any build-up in pressure in the chamber or any excess lowering of temperature near the top such that the dropper system is frozen over time. This exhaust can be controlled manually by venting through an exit pipe which is controlled by a damper. Alternatively, the exhaust gas can be collected under vacuum by the use of an exhaust fan. This cold vapor can be routed to other parts of the process where cold vapors can be utilized such as in storage spaces or with packaging machines.

The first step of the method relates to the preparation of a composition for freezing. The composition may be dairy based and may include such ingredients as cream, milk, butter and/or eggs. In other aspects, the composition may be based on non-dairy ingredients, such as coconut milk, soy milk, or the like, and may comprise ingredients that are less likely to provoke an allergic reaction or aggravate a food sensitivity in a patient. Additional ingredients may include sugar, fruit extracts or some other flavoring component, such as vanilla extract, provided such additional ingredients do not compromise the administration of the therapeutic agent.

After preparing the composition comes the step of slowly dripping the composition into the freezing chamber 12. This may be accomplished in a number of ways. For example, as shown in FIG. 1, the composition C may be pumped from a supply container 30 into a dropper system including a tray 32 positioned across the upper end of the freezing chamber 12. More specifically, the composition is pumped by pump 31 through the tube 33 so as to be delivered through an inlet 35 in the top of the tray that closes the tray to prevent any residual dirt or dust in the air from falling into the composition. The bottom of the tray 32 includes a series of apertures 34 through which the composition drips into the freezing chamber 12. The apertures may have a diameter of between about 3.175 (0.125) to about 7.9375 mm (0.3125 inch) so as to provide the desired size droplets of composition for freezing into beads. The size of the droplets and rate of flow will be determined not only by the size of the holes, but the thickness of the composition and in some cases the thickness of the tray.

As the droplets D of composition fall downwardly in the freezing chamber, they contact cold nitrogen gas rapidly vaporizing from the pool of liquid nitrogen P at the bottom of the chamber. As a result of the temperature within the range of -162.2° (-260°) to -195.5 °C (-320 °F) (for liquid N₂), rapid freezing of the droplets of composition occurs. The small beads B that are produced contain only relatively small ice crystals. The beads B have a smooth, spherical appearance.

An auger 36 for collecting the beads extends into the bottom of the chamber 12. As shown, the auger is positioned at an angle of approximately 45° with respect to the horizontal. The auger may include flights having a diameter of about 3.81 (1.5) to about 8.89 cm (3.5 inches). By rotating such an auger at substantially 10-100 revolutions per minute, it may be possible to complete collecting of the beads from the freezing chamber.

More specifically, as the auger is rotated, the beads B are drawn upwardly in the direction of action arrow E on the flights 38. Liquid refrigerant is, however, not withdrawn from the freezing chamber as sufficient space exists between the flights 38 and the walls of the auger 36 so as to allow the liquid nitrogen to drain back to the pool P. This space is, of course, not large enough to allow the passage of the beads B.

Once the beads B reach the top of the auger 36, they are deposited by means of a chute 40 onto a sieve 42. The sieve 42 is connected to a shaking apparatus 44 as is known in the art. This apparatus 44 serves to vibrate the beads B on the sieve 42. Thus, sifting of the beads B may occur with the relatively large beads having a diameter of, for example, approximately 2 mm or larger remaining on the surface of the sieve while the smaller beads and fragmented portions of broken beads fall through the sieve into the collecting pan 46. That material collected in the pan 46 may, of course, be melted and reprocessed by mixing back in with the composition C that is added to the tray 32 as described above.

The larger beads may flow over the sieve to a discharge chute 48 where they are deposited into a container (not shown). This container may be maintained open for about 1 to about 10 minutes in order to allow any residual nitrogen refrigerant retained in or on the surface of the beads to vaporize. Then the container may be sealed and placed in a freezer for storage.

In order to prevent the beads B from sticking together during storage and thereby maintain their free-flowing character, they must be maintained at a relatively low temperature. More specifically, if the beads B are to be stored for greater than a period of approximately 30 hours, they should be stored in the refrigerator at a temperature of at least as low as -28.9 °C (-20 °F). The beads may be stored at a temperature between -34.4° (-30°) and -40 °C (-40 °F).

Alternatively, if the beads B are to be consumed within a 30-hour period (or shorter period of 10-12 hours for certain compositions), they are to be stored in the freezer at a temperature of -28.9 °C (-20 °F) or above. In one aspect, the beads may be brought to a temperature between about -23.3° (-10°) to about -28.9 °C (-20 °F). In one aspect, the beads may be brought to a temperature of about -26.1 °C (-15 °F). Warmer temperatures may result in the beads sticking together and the product losing its unique free-flowing property which adds to its consumer appeal.

All percentages and ratios are calculated by weight unless otherwise indicated.

All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "20 mm" is intended to mean "about 20 mm."

## Claims

1. A frozen, free-flowing composition comprising a therapeutic agent and at least one flavoring agent for use in therapy, wherein said composition comprises beads comprising an ingredient selected from yogurt, milk, coconut milk, soy milk, almond milk, hazelnut milk, oat milk, hemp milk, and rice milk, and wherein the composition is obtained by a method comprising the steps of:
a) preparing a composition comprising said therapeutic agent for freezing;
b) dripping said composition into a freezing chamber;
c) freezing said dripping composition into beads;
d) collecting said beads from said freezing chamber; and
e) storing said beads at a temperature sufficient to maintain said beads free-flowing for an extended period of time;
wherein said beads are administered after having been stored at a temperature of at least as low as -28.9 °C (-20 °F) and thereafter having been brought to a temperature of from -28.9 °C (-20 °F) to -23.3 °C (-10 °F) before administration such that said beads are free flowing when served; and wherein said beads are administered in an amount such that an effective amount of said therapeutic agent is administered.

2. The composition of claim 1, wherein the temperature within said freezing chamber is kept below -162.2 °C (-260 °F).

3. The composition of claims 1 and 2, wherein the method comprises the step of dripping said composition through a tray having a series of apertures having diameters of predetermined sizes.

4. The composition of any of the preceding claims, wherein the beads are collected from said freezing chamber by utilizing an auger.

5. The composition of claim 4, wherein said auger is positioned at approximately 45° relative to horizontal and includes flights having a diameter of 3.81 (1.5) to 8.89 cm (3.5 inches) and said collecting step includes the step of rotating said auger at 10 to 100 revolutions per minute.

6. The composition of any of the preceding claims, wherein storing said beads at a temperature sufficient to maintain said beads free-flowing for an extended period of time is at a temperature of from -32°C to -40°C.

7. The composition of any of the preceding claims, wherein said beads have a diameter of between 2 mm and 10 mm.

8. The composition of any of the preceding claims, wherein said beads have a diameter from 3.17 (0.125) to 7.937 mm (0.3125 inch).

9. The composition of any of the preceding claims, wherein liquid nitrogen is utilized to freeze said composition and the method further includes the step of maintaining said container open for a sufficient period of time to allow any nitrogen retained in said beads during freezing to vaporize.

10. The composition of any of the preceding claims, wherein said beads are brought to a temperature of -26.1 °C (-15 °F) prior to serving.

11. The composition of any of the preceding claims, including a step of sifting beads collected from said freezing chamber, wherein beads having a diameter larger than 2 mm during sifting are recovered.

12. The composition of any of the preceding claims, wherein said therapeutic agent comprises a therapeutic agent suitable for treating one or more allergy symptoms selected from diphenhydramine, loratadine, fexofenadine, cetirizine, montelukast, and combinations thereof.

13. The composition of claims 1 to 11, wherein said therapeutic agent comprises prednisone.

14. The composition of any of the preceding claims, wherein said composition contains less than 0.1 % by weight of any one or more of dairy, nuts, sugar, and gluten.

## Patentansprüche

1. Gefrorene, frei fließende Zusammensetzung, enthaltend einen therapeutischen Wirkstoff und mindestens einen Geschmacksstoff zur Verwendung in der Therapie, wobei diese Zusammensetzung Perlen enthaltend einen Inhaltsstoff ausgewählt von Joghurt, Milch, Kokosmilch, Sojamilch, Mandelmilch, Haselnussmilch, Hafermilch, Hanfmilch und Reismilch enthält und wobei die Zusammensetzung erhalten wird durch ein Verfahren, umfassend die Schritte:
a) Herstellen einer Zusammensetzung enthaltend den therapeutischen Wirkstoff zum Einfrieren;
b) Tropfen der Zusammensetzung in eine Gefrierkammer;
c) Einfrieren der tropfenden Zusammensetzung in Perlen;
d) Entnehmen der Perlen aus der Gefrierkammer; und
e) Lagern der Perlen bei einer Temperatur, welche ausreichend ist, die Perlen über einen längeren Zeitraum frei fließend zu erhalten;
wobei die Perlen verabreicht werden, nachdem sie bei einer Temperatur von mindestens so tief wie -28,9°C (-20°F) gelagert wurden und danach vor Verabreichung auf eine Temperatur von -28,9°C (-20F) bis -23,3°C (-10°F) gebracht wurden, so dass die Perlen frei fließend sind, wenn sie serviert werden; und
wobei die Perlen in einer Menge verabreicht werden, so dass eine effektive Menge des therapeutischen Wirkstoffs verabreicht wird.

2. Zusammensetzung nach Anspruch 1, wobei die Temperatur in der Gefrierkammer unter -162,2°C (-260°F) gehalten wird.

3. Zusammensetzung nach Ansprüchen 1 und 2, wobei das Verfahren den Schritt des Tropfens der Zusammensetzung durch eine Platte aufweisend eine Serie von Öffnungen aufweisend einen Durchmesser von vorbestimmter Größe umfasst.

4. Zusammensetzung nach einem der vorhergehenden Ansprüche, wobei die Perlen aus der Gefrierkammer unter Verwendung einer Förderschnecke entnommen werden.

5. Zusammensetzung nach Anspruch 4, wobei die Förderschnecke ungefähr 45° relativ zur Horizontalen positioniert ist und Flügel aufweisend einen Durchmesser von 3,81 (1,5) bis 8,89 cm (3,5 inch) umfasst und der Entnahmeschritt den Schritt der Rotation der Förderschnecke bei 10 bis 100 Umdrehungen pro Minute umfasst.

6. Zusammensetzung nach einem der vorhergehenden Ansprüche, wobei Lagern der Perlen bei einer Temperatur ausreichend, um die Perlen für einen ausgedehnten Zeitraum frei fließend zu erhalten, eine Temperatur von -32°C bis -40°C ist.

7. Zusammensetzung nach einem der vorhergehenden Ansprüche, wobei die Perlen einen Durchmesser von zwischen 2 mm und 10 mm aufweisen.

8. Zusammensetzung nach einem der vorhergehenden Ansprüche, wobei die Perlen einen Durchmesser von 3,17 (0,125) bis 7,937 mm (0,3125 inch) aufweisen.

9. Zusammensetzung nach einem der vorhergehenden Ansprüche, wobei flüssiger Stickstoff verwendet wird, um die Zusammensetzung einzufrieren und das Verfahren weiterhin den Schritt des Offenhaltens des Behälters für einen Zeitraum, welcher ausreichend ist, um das Verdampfen von jeglichem Stickstoff, welcher in den Perlen während des Einfrierens gespeichert wurde, zu ermöglichen.

10. Zusammensetzung nach einem der vorhergehenden Ansprüche, wobei die Perlen vor dem Servieren auf eine Temperatur von -26,1°C (-15°F) gebracht werden.

11. Zusammensetzung nach einem der vorhergehenden Ansprüche, enthaltend einen Schritt des Siebens von Perlen, welche aus der Gefrierkammer entnommen werden, wobei Perlen aufweisend einen Durchmesser von größer als 2 mm während des Siebens gewonnen werden.

12. Zusammensetzung nach einem der vorhergehenden Ansprüche, wobei der therapeutische Wirkstoff einen therapeutischen Wirkstoff umfasst, welcher zur Behandlung von einem oder mehrerer allergischer Symptome geeignet ist, ausgewählt aus Diphenylhydramin, Loratadin, Fexofenadin, Cetrizin, Montelukast und Kombinationen dieser.

13. Zusammensetzung nach Ansprüchen 1 bis 11, wobei der therapeutische Wirkstoff Prednison enthält.

14. Zusammensetzung nach einem der vorhergehenden Ansprüche, wobei die Zusammensetzung weniger als 0,1 Gew.-% von einem oder mehreren von Milchprodukten, Nüssen, Zucker und Gluten enthält.

## Revendications

1. Composition congelée à écoulement libre comprenant un agent thérapeutique et au moins un agent aromatique pour son utilisation en thérapie, dans laquelle ladite composition comprend des billes comprenant un ingrédient sélectionné parmi le yaourt, le lait, le lait de coco, le lait de soja, le lait d'amande, le lait de noisette, le lait d'avoine, le lait de chanvre et le lait de riz, et dans laquelle la composition est obtenue par une méthode comprenant les étapes :
a) de préparation d'une composition comprenant ledit agent thérapeutique pour la congélation ;
b) d'égouttage de ladite composition dans une chambre de congélation ;
c) de congélation de ladite composition s'égouttant en billes ;
d) de collecte desdites billes de ladite chambre de congélation ; et
e) de stockage desdites billes à une température suffisante pour maintenir l'écoulement libre desdites billes pendant une période de temps prolongée ;
dans laquelle lesdites billes sont administrées après avoir été stockées à une température au moins aussi basse que -28,9 °C (-20 °F) puis, après ceci, avoir été portées à une température de -28,9 °C (-20 °F) à -23,3 °C (-10 °F) avant l'administration de telle sorte que les billes s'écoulent librement lorsqu'elles sont servies ; et
dans laquelle lesdites billes sont administrées en une quantité telle qu'une quantité efficace dudit agent thérapeutique est administrée.

2. Composition selon la revendication 1, dans laquelle la température à l'intérieur de ladite chambre de congélation est maintenue au-dessous de -162,2 °C (-260 °F).

3. Composition selon les revendications 1 et 2, dans laquelle la méthode comprend l'étape d'égouttage de ladite composition à travers un plateau ayant une série d'ouvertures ayant des diamètres de tailles prédéterminées.

4. Composition selon l'une quelconque des revendications précédentes, dans laquelle les billes sont collectées de ladite chambre de congélation à l'aide d'une vis sans fin.

5. Composition selon la revendication 4, dans laquelle ladite vis sans fin est positionnée à approximativement 45° par rapport à l'horizontale et comprend des déflecteurs ayant un diamètre de 3,81 (1,5) à 8,89 cm (3,5 pouces) et ladite étape de collecte comporte l'étape de mise en rotation de ladite vis sans fin à 10 à 100 tours par minute.

6. Composition selon l'une quelconque des revendications précédentes, dans laquelle le stockage desdites billes à une température suffisante pour maintenir l'écoulement libre des billes pendant une période de temps prolongée est réalisé à une température de -32 °C à -40 °C.

7. Composition selon l'une quelconque des revendications précédentes, dans laquelle lesdites billes ont un diamètre compris entre 2 mm et 10 mm.

8. Composition selon l'une quelconque des revendications précédentes, dans laquelle lesdites billes ont un diamètre de 3,17 (0,125) à 7,937 mm (0,3125 pouces).

9. Composition selon l'une quelconque des revendications précédentes, dans laquelle de l'azote liquide est utilisé pour congeler ladite composition et ladite méthode comprend en outre l'étape de maintien dudit récipient ouvert pendant une période de temps suffisante pour permettre que tout l'azote retenu dans lesdites billes s'évapore.

10. Composition selon l'une quelconque des revendications précédentes, dans laquelle lesdites billes sont portées à une température de -26,1 °C (-15 °F) avant d'être servies.

11. Composition selon l'une quelconque des revendications précédentes, comportant une étape de tamisage des billes collectées de ladite chambre de congélation, dans laquelle les billes ayant un diamètre supérieur à 2 mm durant le tamisage sont récupérées.

12. Composition selon l'une quelconque des revendications précédentes, dans laquelle ledit agent thérapeutique comprend un agent thérapeutique adapté pour le traitement d'un ou plusieurs symptômes allergiques sélectionnés parmi la diphenhydramine, la loratadine, la fexofénadine, la cétirizine, le montélukast et les combinaisons de ceux-ci.

13. Composition selon les revendications 1 à 11, dans laquelle ledit agent thérapeutique comprend de la prednisone.

14. Composition selon l'une quelconque des revendications précédentes, dans laquelle ladite composition contient moins de 0,1 % en poids de l'un quelconque ou plus d'un produit laitier, de noix, de sucre et de gluten.
